# EUROPEAN PATENT APPLICATION

(11) **EP 4 137 184 A1**
(43) Date of publication of application: **22.02.2023**
(21) Application number: 21191536.8
(22) Date of filing: 16.08.2021
(51) Int. Cl.: A61M 15/00, G16H 20/00, A61B 5/00, G06K 19/067, G16H 40/63, G06K 7/10

(54) **ADHERENCE / COMPLIANCE MONITOR FOR AN INHALER**

(71) Applicant: Vectura Delivery Devices Limited, Chippenham, Wiltshire SN14 6FH (GB)
(72) Inventor: THOMAS, Seth, Cambridge, CB4 0GZ (GB)
(74) Representative: Clarke, Christopher John

(57) **Abstract**

A monitor (300) for an inhaler is provided which comprises one or more sensors (320), a microcontroller (310) which is configured to process and/or store usage data determined from the sensor(s) relating to a patient's usage of the inhaler, a battery (330) and a wireless communication unit (340) having at least one antenna (360) for transmitting the usage data to an external device via electromagnetic coupling. The monitor is characterized by the wireless communication unit comprising an RFID controller (350) communicatively coupled to the microcontroller (310) via a synchronous serial communication bus for allowing the microcontroller to communicate the usage data to the RFID controller (350), the RFID controller (350) having a memory (355) for storing the received usage data for wireless transmission to the external device.

## Description

### Technical Field of the Invention

The present invention relates to an inhaler containing an active substance for inhalation and a monitor having one or more sensors for monitoring a patient's adherence and / or compliance. In particular, the invention relates to a monitor with improved battery power management.

### Background to the Invention

Inhalers, such as dry powder inhalers (DPIs), provide an attractive method for administering medicaments, for example to treat local diseases of the airway or to deliver drugs to the bloodstream via the lungs. The medicament is commonly provided as individual doses, such as a strip having a plurality of blisters, for example as disclosed in WO13/175177. The efficacy of treatment is dependent on the patient using the inhaler correctly and as prescribed. Consequently, there is increasing interest in monitoring patient adherence, i.e. whether the patient takes the prescribed number of doses per day, e.g. once or twice daily and compliance, i.e. whether the patient uses the inhaler correctly, e.g. inhales at the correct rate.

Monitors have been developed that provide adherence and / or compliance information. Such monitors contain sensors, electronics and a power supply, such as a battery, and may be built-in to the inhaler, or may be a separate unit that is attachable to the inhaler. They typically determine when the inhaler has been used (e.g. by detecting actuation optically, acoustically or by switches) and measure the effectiveness of the inhalation (e.g. by measuring the air pressure in the mouthpiece and determining the flow rate, duration of inhalation etc.). This information is often then transmitted wirelessly, e.g. by Bluetooth^{®}, to an external device, such as a smartphone, which records and / or displays it, in order to provide feedback to the patient and / or a care giver.

WO 2019/010197 discloses an inhaler device with electrodes arranged on the mouthpiece, to detect when an individual places her lips on the mouthpiece. The inhaler device may only allow medication to be administered upon receiving an indication that a contact event has been recorded by some or all of the electrode(s). The device has a communication module that is able to send and/or receive data over wireless communication protocols, such as Wi-Fi, Bluetooth^{®}, Near Field Communication (NFC), radio-frequency identification (RFID), or wireless Universal Serial Bus (USB). WO2019/209994 discloses a monitor for an inhaler having an RFID reader for reading an RFID tag in a cartridge loaded in the inhaler, and a wireless transceiver, such as Bluetooth^{®} over which data received from the at least two sensors is transmitted to a remote processing system.

Power management is typically a concern for such monitors. They may not be used until several months after manufacture, and the battery then needs to last for at least the expected life of the inhaler (typically one month or more). The size of the monitor is limited (in order that it does not interfere with the use of the inhaler), so the size, and hence the capacity, of the battery is also limited. In order to minimize power consumption, the monitor typically enters a low power (sleep) state between uses, and may be configured to be in a fully off state prior to its first use, as disclosed in WO 2018/104268. Nonetheless, there remains a need for further improvements in the power use in such monitors, in order to extend their lifetime.

### Brief description of the invention

In a first aspect, the invention provides a monitor for an inhaler, wherein the monitor comprises one or more sensors, a microcontroller which is configured to process and/or store usage data determined from the sensor(s) relating to a patient's usage of the inhaler, a battery and a wireless communication unit having at least one antenna for transmitting the usage data to an external device via electromagnetic coupling, characterized by the wireless communication unit comprising an RFID controller communicatively coupled to the microcontroller via a synchronous serial communication bus for allowing the microcontroller to communicate the usage data to the RFID controller, the RFID controller having a memory for storing the received usage data for wireless transmission to the external device.

The synchronous serial communication bus may be a bus according to an I2C protocol. I2C is a wired protocol which allows the microcontroller to write data to the RFID controller, without requiring a separate RFID writer. The RFID controller comprises, or is associated with, a passive antenna and a memory device. It is activated by an RFID transponder on the external device, such as a smart phone. Once activated, data stored in the memory of the RFID controller may be read by the RFID transponder using electromagnetic coupling, and delivered to an app on the external device for analysis and / or display. Thus the monitor does not need a separate a wireless communicator such as Bluetooth^{®}.

This can provide a significant power saving for two reasons: firstly, in contrast to a Bluetooth^{®} wireless communicator, there is no need to have a permanently open communication channel, which is a significant drain on the battery. Secondly, the power required for data transmission is supplied from the external device by the RFID transponder to the communication unit of the monitor by means of inductive or electromagnetic coupling. Thus the wireless communicator does not drain power from the battery in the monitor, so its lifetime is extended.

The RFID controller may be configured to harvest energy from the external device via the antenna when the external device is present i.e. whilst electromagnetically coupled. The microcontroller may be configured to pass surplus power from the external device to the microcontroller and / or to the battery.

The microcontroller may be configured to determine whether the external device is present, and, if the external device is present, to transmit the data from the RFID controller to the external device and to perform one or more further tasks. The further task(s) may comprise compressing or trimming data before, during or after the data transfer, clearing the data in the RFID controller in preparation for receiving further data, and / or cueing up the next usage data for transmission.

The one or more sensors may include optical sensor(s), such as photomicrosensors which read codes on the blister strip, or which detect movement of components of the inhaler; air pressure sensors which detect the pressure during inhalation; pressure sensors, which detect actuation from the pressure of the user's fingers on part of the inhaler; proximity sensors, such as electrodes or temperature sensors which detect the presence of a part of the user's body, such as the presence of lips at the mouthpiece; microphones which detect audible sounds that are indicative of actuation and / or inhalation; acceleration sensors which detect motion of the monitor, such as when it is picked up by the user, and / or the orientation of the device; switches, such as mechanical switches, or magnetic reed switches, which can detect motion of a part of the inhaler, such as removal or opening of a mouthpiece cover; electrical contacts or any other suitable type of sensor.

In one embodiment, the monitor has a barometric pressure sensor for measuring the pressure during inhalation, and the further task(s) comprise taking a tare measurement from the pressure sensor.

In a second aspect, the present invention provides a kit or a combination comprising a monitor of the first aspect and an inhaler, such as a dry powder inhaler which contains a blister strip comprising a plurality of blisters. The monitor may be removably attachable to the inhaler, or permanently attached to the inhaler.

The inhaler may have a mechanism for advancing the blister strip and for opening the blisters which is operated by an actuator. The opening mechanism may be a piercer which is mounted on the underside of the mouthpiece. The actuator may drive the indexing mechanism to move one or more blisters into alignment with the piercer and then move the mouthpiece relative to the housing so that the piercer pierces the aligned blister(s). The actuator may be a lever which causes indexing of the blister strip and piercing of the blisters. Alternatively, the actuator may be formed as part of, or be connected to, the mouthpiece cover, so that rotation of the cover causes indexing of the blister pack and piercing of the blisters. The inhaler may be of the type described in WO13/175177 which has a blister strip that is advanced by pivoting the mouthpiece cover: in a first stage, moving the cover from the closed position to an intermediate position causes the blister strip to be advanced; and in a second stage, moving the cover from the intermediate position to the open position operates the piercer.

### Brief Description of the Figures

The invention will now be further described with reference to the Figures, wherein:
Figure 1A shows an inhaler and a monitor according to the invention, with the cover in the closed position, so that the mouthpiece is covered.
Figure 1B shows the inhaler of Figure 1A with the cover in the open position so that the mouthpiece is exposed.
Figure 1C shows another view of the inhaler of Figure 1A, from a different angle.
Figure 1D shows the inhaler of Figure 1A with the monitor removed and the cover closed.
Figures 2A and 2B show the monitor removed from the inhaler.
Figure 3 schematically illustrates a monitor according to the invention.

### Detailed Description of the Invention

In the context of dry powder inhalers, the term "adherence" is normally used to refer to whether the patient takes the prescribed number of doses per day, e.g. once or twice daily. The term "compliance" is normally used to refer to whether the patient uses their inhaler correctly, e.g. if they inhale sufficiently strongly to entrain the powder and disperse it into particles that reach the lung. Consequently, a monitor may be designed to measure adherence and / or compliance, according to the type of sensors that it uses, and how they are configured. In the present application, the term "monitor" therefore refers to a module having one or more sensors that is designed to measure and capture information relating to adherence, and / or compliance. The monitor does not perform any of the functions associated with dosing the medication, such as a piercing or opening blisters, deagglomerating the powder or providing a breath-actuation mechanism. The inhaler therefore operates to dispense powder whether the monitor is present or not.

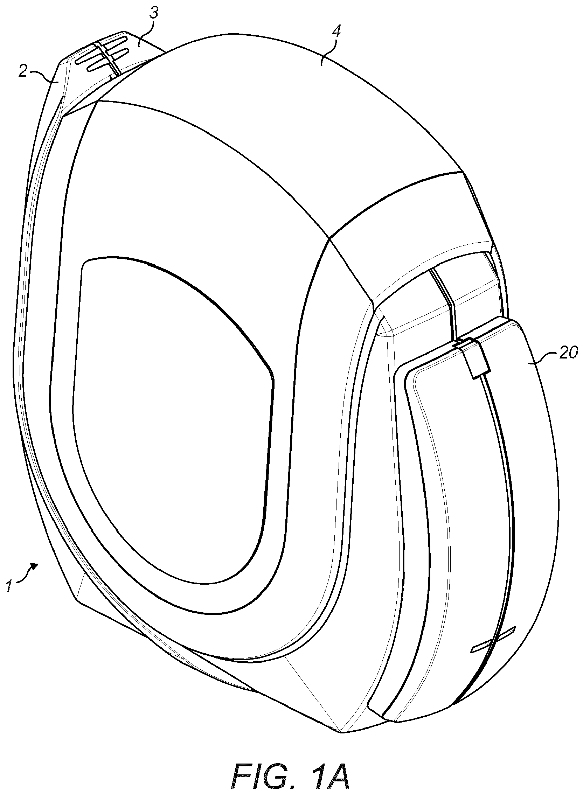

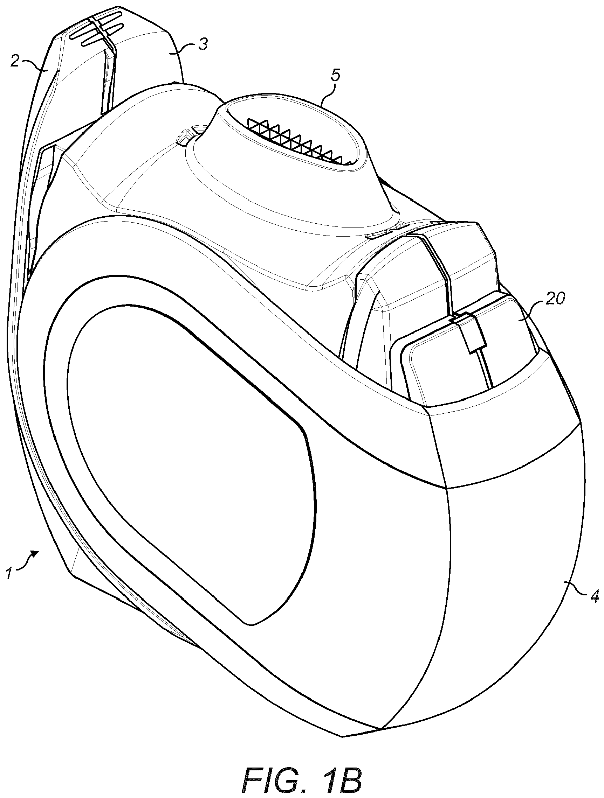

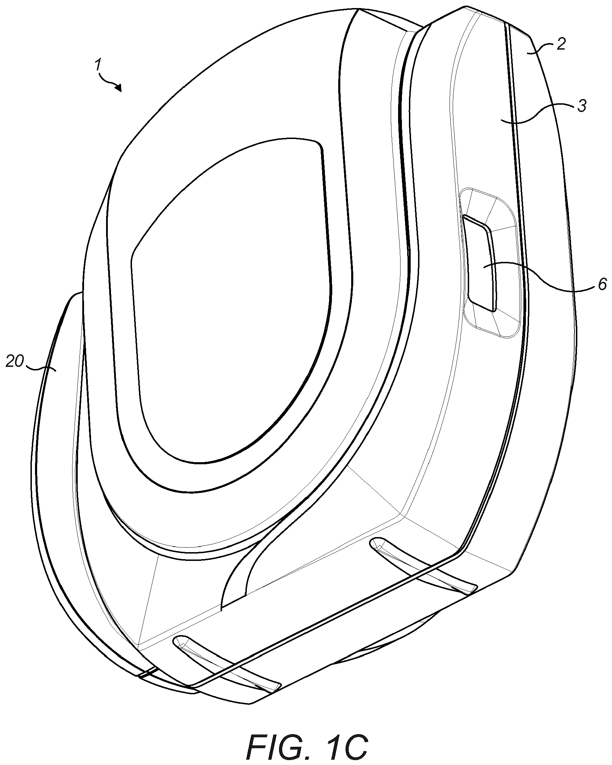

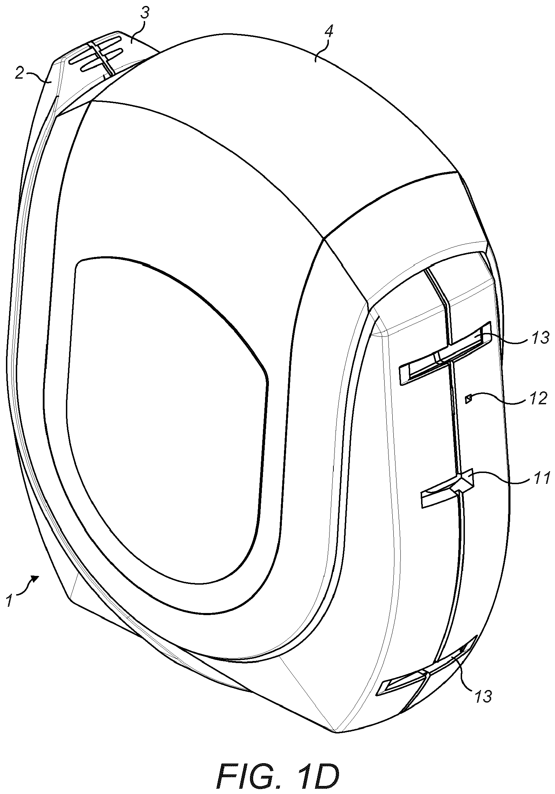

An inhaler and monitor according to the invention are shown in Figure 1. Figures 1A and 1C show an inhaler with a monitor attached from two different angles, with the mouthpiece cover in the closed position. Figure 1B shows the inhaler and monitor with the mouthpiece cover in the open position so that the mouthpiece is visible. Figure 1D shows the inhaler with the monitor removed and the cover closed.

The inhaler shown in Figure 1 is an "open-inhale-close" inhaler of the type described in WO13/175177. However, the invention is not limited to this type of inhaler, and for example, could equally be used with an inhaler which has a passive mouthpiece cover, and a separate actuating lever, as described for example in WO13/175176.

The inhaler **1** shown in Figure 1A is constructed from two shell parts **2, 3** which are joined together to form a housing that contains a blister strip. A detachable monitor **20** is attached to one side of the inhaler. A mouthpiece cover **4** is mounted onto the housing. The cover **4** can be rotated (through an angle somewhat greater than 90°) from the closed position (Figure 1A) in which it covers and protects a mouthpiece **5** to a fully open position (Figure 1B), in which the mouthpiece **5** is exposed so that the user can inhale a dose of medicament.

Figure 1C shows a view of the inhaler of Figure 1A, from a different angle. The housing has an opening **6** on the opposite side from the monitor **20.** This allows the user to see a number printed on the blister strip. When the user actuates the inhaler by opening the cover, the indexing mechanism advances the blister strip by one dose. As a result, the number on the blister strip that is visible to the user sequentially decreases (or increases). The numbers therefore provide a dose counter which displays the number of doses remaining (or that remain to be dispensed) in the inhaler.

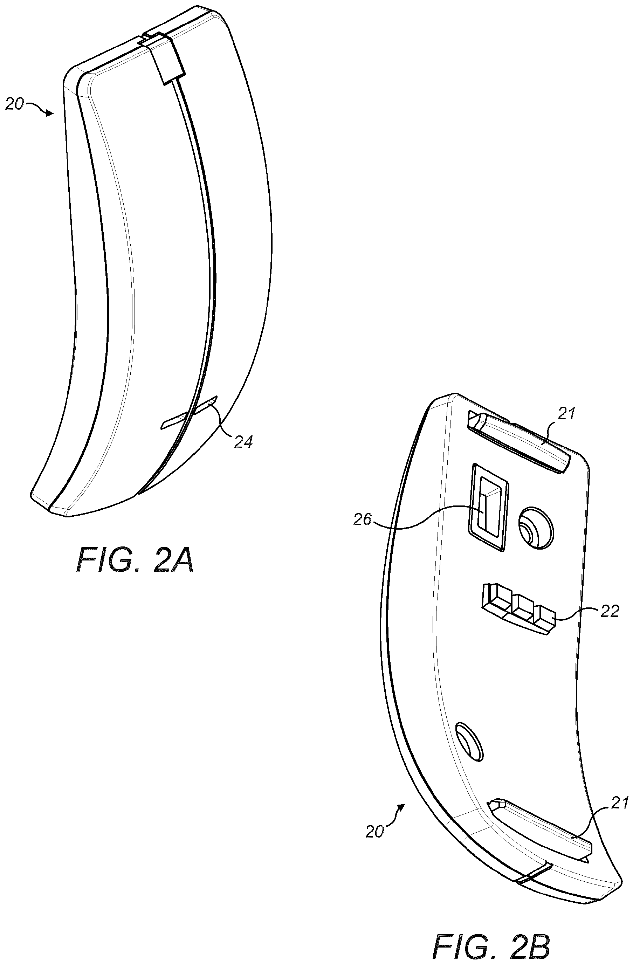

Figure 1D shows the inhaler with the monitor having been removed. An aperture **11** is visible in the wall of the housing where the monitor was attached. The aperture allows the monitor to read indicia on the blister strip. Instead of having an aperture, a portion of the housing may be transparent such that the indicia are visible externally. There is also a small orifice **12** in the wall of the housing, which allows a pressure sensor **26** (shown in Figure 2B) in the monitor to connect to the mouthpiece via a channel inside the inhaler. The housing also has two slots **13** for mounting the monitor as described below.

The inhaler has a strip of blisters containing powdered medicament for inhalation. The blister strip is typically cold-formed from a ductile foil laminate or a plastics material and includes a pierceable lid, typically foil or a foil laminate (e.g. aluminium), which is heat-sealed around the periphery of the blister after the dose of medicament has been introduced during manufacture.

The mouthpiece **5** is formed as part of a component which is pivotally mounted to the housing. A piercer is located directly underneath the mouthpiece. The cover is selectively coupled to a blister strip indexing mechanism and to the mouthpiece component. Moving the cover from the closed position to an intermediate position (the first stage of opening) causes the indexing mechanism to advance the blister strip. Then, once an unused blister is in position beneath the piercer, the indexing mechanism is disengaged. The first stage is reversible, i.e. the user can abort the actuation of the inhaler (since piercing has not yet occurred) simply by closing the cover, which moves the blister strip back to its previous position. Moving the cover from the intermediate position to the fully open position (the second stage) causes the mouthpiece component to pivot downwards so that the piercer pierces the aligned blister. The user then inhales through the mouthpiece, which aerosolizes the powder in the pierced blister.

The inhaler may be configured to index and pierce one blister on each actuation. Alternatively, it may index and pierce two (or more) blisters on each actuation, and thereby deliver two (or more) different formulations or medicaments simultaneously. Alternatively, it may for example deliver a large amount of the medicament, e.g. double or triple, by piercing two or three blisters. In other words, a dose of medicament may be provided by one blister, or by more than one blister, for example two blisters with different medicaments which are delivered in a single actuation.

The medicament is suitable for administration by inhalation, for example for the treatment of a respiratory disease. It may include one of more of the following classes of pharmaceutically active material: anticholinergics, adenosine A2A receptor agonists, β2-agonists, calcium blockers, IL-13 inhibitors, phosphodiesterase-4-inhibitors, kinase inhibitors, steroids, CXCR2, proteins, peptides, immunoglobulins such as Anti-IG-E, nucleic acids in particular DNA and RNA, monoclonal antibodies, small molecule inhibitors and leukotriene B4 antagonists. The medicament may include excipients, such as fine excipients and / or carrier particles (for example lactose), and / or additives (such as magnesium stearate, phospholipid or leucine).

Suitable β2-agonists include albuterol (salbutamol), preferably albuterol sulfate; carmoterol, preferably carmoterol hydrochloride; fenoterol; formoterol; milveterol, preferably milveterol hydrochloride; metaproterenol, preferably metaproterenol sulfate; olodaterol; procaterol; salmeterol, preferably salmeterol xinafoate; terbutaline, preferably terbutaline sulphate; vilanterol, preferably vilanterol trifenatate or indacaterol, preferably indacaterol maleate.

Suitable steroids include budesonide; beclamethasone, preferably beclomethasone dipropionate; ciclesonide; fluticasone, preferably fluticasone furoate; mometasone, preferably mometasone furoate.

Suitable anticholinergics include: aclidinium, preferably aclidinium bromide; glycopyrronium, preferably glycopyrronium bromide; ipratropium, preferably ipratropium bromide; oxitropium, preferably oxitropium bromide; tiotropium, preferably tiotropium bromide; umeclidinium, preferably umeclidinium bromide; Darotropium bromide; or tarafenacin.

The active material may include double or triple combinations such as salmeterol xinafoate and fluticasone propionate; budesonide and formoterol fumarate dihydrate; glycopyrrolate and indacaterol maleate; glycopyrrolate, indacaterol maleate and mometasone furoate; fluticasone furoate and vilanterol; vilanterol and umeclidinium bromide; fluticasone furoate, vilanterol and umeclidinium bromide.

Figure 2 shows the monitor **20** on its own (i.e. detached from the inhaler). Figure 2A shows the outer side of the monitor, and Figure 2B shows the inside face of the monitor (i.e. the side which abuts the inhaler when the monitor is attached). The monitor **20** has two clips **21** which fit into the corresponding slots **13** in the housing, and thereby hold the monitor in place when attached to the inhaler. The clips and slots allow the monitor to be detachably mounted on the inhaler, e.g. by an interference fit. A detachable monitor has the advantage that it may be supplied separately from the inhaler, and that a single monitor may be used with many different inhalers. Thus, when the medication in the inhaler has been used up, the monitor can be detached and then re-attached to a new inhaler. The new inhaler could be identical to the used one, or it could contain a different dose strength or a different active, or even be a different type of inhaler, provided that it is compatible with the monitor. Alternatively, the monitor may be intended for use with a single inhaler only, in which case it may be permanently attached to the inhaler, e.g. by ultrasonic welding or glueing.

The monitor 20 has a power source, such as a rechargeable battery. The monitor 20 may have a motion sensor, such as an accelerometer, and means for switching on the monitor when motion is detected. The motion sensor may be configured to sense a specific gesture, such as picking up the monitor. Alternatively, a reed switch and a corresponding magnet on the mouthpiece cover, or a mechanical switch which interacts with the cover can be used to switch the monitor on. This avoids the need for the monitor to be permanently switched on, and hence conserves battery power.

The monitor 20 has a microcontroller and memory (e.g. a suitable microprocessor) which are configured to process and/or store information read from the sensors and / or switches relating to patient's usage of the inhaler. The monitor has a wireless communicator for transmitting data to an external device, such as a computer or smartphone. The wireless communicator is an RFID controller, which is described in further detail below. The information may then be displayed to the user and / or a medical professional, by means of suitable software, for example a smartphone app. The information may additionally or alternatively be stored on the monitor for subsequent interrogation, and / or transmitted to an online health platform. The wireless communicator may also receive information from the external device.

The monitor may have a pressure sensor **26,** which is located in a recess on the inside face (see Figure 2B). The pressure sensor abuts the orifice **12** in the housing (see Figure 1D), which leads via a channel in the inhaler housing to the mouthpiece. Alternatively, the pressure sensor could be connected to the mouthpiece via a separate external tube. The monitor can thereby measure the pressure in the mouthpiece to sense the user's inhalation. The pressure sensor may be a differential pressure sensor, or a barometric (absolute) pressure sensor.

The monitor may have one or more optical sensors **22** for reading the indicia on the blister strip via the aperture **11.** In the embodiment shown in Figure 2, the monitor has three photomicrosensors, each consisting of a light source, such as a light emitting diode (LED) and a photodetector with an attached light guide. The light guide channels light from the LED to the blister strip where it is reflected back through the light guide to the photodetector. The monitor uses the reflected light signals to read a code on the blister strip. It can then determine the number of the current blister, so that the number of doses that have been dispensed or that remain to be dispensed can be read by the monitor from the blister strip.

Previously known monitors typically use Bluetooth^{®} communications in order to relay information to an external device (e.g. a smart phone) for processing, recording and / or display. However Bluetooth (and the like) requires power from the battery of the monitor, which reduces the lifetime of the monitor which is typically a sealed unit preventing battery replacement. The present inventor has identified that a monitor may be provided with a communication unit comprising a passive RFID interface with a synchronous serial communication bus, such as an I2C interface, which allows the microcontroller to write data to be transmitted to a memory of the RFID interface.

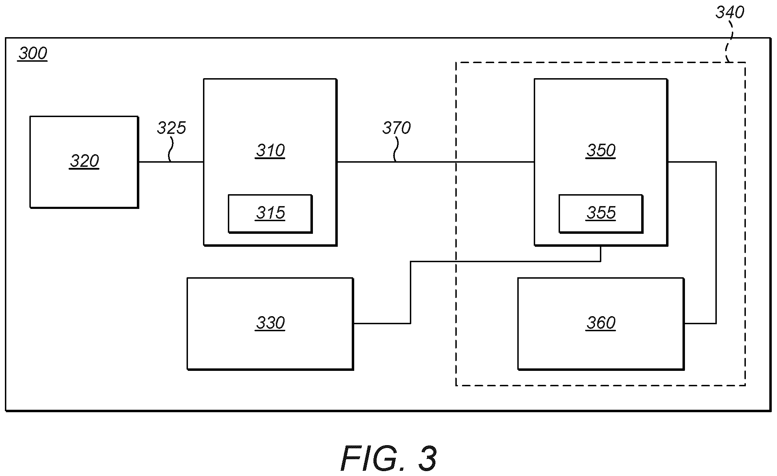

Referring to Figure 3, a monitor 300 according to an embodiment of the present invention is schematically illustrated. The monitor 300 comprises a microcontroller 310 having an associated memory 315 for storing data therein, one or more sensors 320 as discussed above and a battery 330 for storing energy to power at least the microcontroller 310. The one or more sensors 320 are arranged to measure one or more parameters relating to a patient's usage of the inhaler associated with the monitor 300 and to output one or more signals 325 indicative thereof to the microcontroller 310. The microcontroller 310 is arranged to process the one or more signals 325 and to store data in the memory 315 indicative of the patient's usage of the inhaler. It should be noted that whilst the memory 315 is shown as forming part of the microcontroller 310 in Figure 3, the memory 315 may be external to the microcontroller 310 in some embodiments.

The monitor 300 further comprises a communication unit 340 for wirelessly transmitting data indicative of the patient's usage of the inhaler, hereinafter referred to as usage data. The communication unit 340 provides a passive RFID interface for wirelessly transmitting the data to an RFID transponder of an external device. The communication unit 340 comprises an RFID controller 350 and at least one antenna 360. The RFID controller 350 is associated with a respective memory 355 for storing the usage data to be wirelessly transmitted, which is received from the microcontroller 310 as will be explained. The passive nature of the RFID interface advantageously reduces power consumption of the monitor 300 communicating the usage data. The battery 330 is illustrated in Figure 3 as being connected to the RFID controller 350; nonetheless, the battery 330 may also provide power to other components of the monitor 300, in particular the microcontroller 310.

A wired communication interface 370 is provided between the microcontroller 310 and the RFID controller 350. The wired communication interface 370 is a synchronous serial data interface between the microcontroller 310 and the RFID controller 350. Advantageously, the synchronous serial data interface allows usage data prepared by the microcontroller 310 to be efficiently communicated to the RFID controller 350 and stored in the memory 355 associated with the RFID controller 350, such that the usage data stored therein is ready for wireless transmission when the at least one antenna 360 is energised by the RFID transponder of the external device.

In some embodiments, the synchronous serial data interface is an I2C (also known as I²C, IIC or Inter-Integrated Circuit) communication bus 370. I2C is a synchronous, multi-master, multi-slave, packet-switched, single-ended, serial communication bus specification. As explained above, an I2C bus 370 allows the microcontroller 310 to write data to the RFID controller 350, without requiring a separate RFID writer (which is typically used for RFID interfaces that are only intended to be read).

In use, when the external device (such as a laptop computer, tablet computer or mobile phone having the RFID transponder) is brought close to the monitor 300, an antenna of the RFID transponder and the at least one antenna 360 of the communication unit 340 become electromagnetically coupled. The at least one antenna 360 of the monitor 300 is powered by energy from radio waves transmitted by the RFID transponder of the external device. The RFID controller 350 is able to modulate the usage data stored in the memory 355 onto the electromagnetic coupling for wireless transmission to the external device.

The usage data communicated to the external device is delivered to software executing thereon, such as an app on the external device, for analysis and / or display. Thus the monitor 300 does not need a powered or active wireless communicator such as Bluetooth^{®} which advantageously reduces energy consumption.

Data provided by the RFID controller 350 may be encrypted and may then be decrypted by the external device via a decryption key contained within the memory 355.

In some embodiments, when the external device is electromagnetically coupled to the at least one antenna 360 of the communication unit 340 and electrical energy induced therein, the RFID controller 350 is arranged to harvest a portion of the electrical energy. The harvested energy may be used to power one or more sub-systems of the monitor 300. In some embodiments, the RFID controller 350 is arranged to direct at least some of the harvested electrical energy to the battery 330 for recharging the battery. In this way, the lifetime of the monitor may be increased by the energy harvesting.

Using an I2C RFID interface instead of a wireless communicator, such as a Bluetooth^{®} antenna can provide a significant power saving for several reasons.

Firstly, unlike a Bluetooth^{®} wireless communicator, there is no need to have a permanently open communication channel, which is a significant drain on the battery.

Secondly, the at least one antenna 360 of the RFID interface can (unlike a Bluetooth^{®} antenna) harvest energy from the external device by means of electromagnetic or inductive coupling. This energy can be used to run low power devices, such as the microcontroller 310. Thus the power required for data transmission is supplied from the external device by the RFID transponder to the RFID interface of the monitor 300. Consequently, the wireless communication unit 340 does not drain power from the battery 330 in the monitor 300, so its lifetime is extended. The microcontroller 310 can also be configured to pass any surplus power from the external device to the battery 330, and hence partially re-charge it.

Thirdly, when the external device is close to the monitor 300, the electromagnetic field from the transponder is detected by the antenna 360 and the RFID controller 350. From this, the microcontroller 310 is able to determine that the external device is present, such as via signalling from the RFID controller 350 to the microcontroller 310 via the wired interface 370. Alternatively, such signalling may be performed by a dedicated line, such as a 'field detected' signalling line between the RFID controller 350 and the microcontroller 310. Some of the tasks of the microcontroller 310 can therefore be timed to occur during, or immediately before, or after, data transmission. By doing so, the power required for these tasks can be supplied by the external device rather than the battery 330 of the monitor 300, thus further reducing the battery drain on the monitor 300.

For example, the microcontroller 310 may be arranged to compress or trim the data (for example by removing near-zero values from the beginning and end of a pressure difference measurement during inhalation). The data capacity of the memory 355 associated with the RFID controller 350 may be fairly limited, so this would be advantageous in order to ensure that the usage data fits within the memory 355 of the RFID controller 350 , especially if the monitor 300 had not uploaded to or synched with the external device after the previous treatment.

Another possibility, once the presence of the external device has been detected, is that the microcontroller 310 could assume that the current dataset has been uploaded, and then delete this uploaded dataset from the memory 355 of the RFID controller 350 to free up memory space. Alternatively or additionally, the microcontroller may cue up the next dataset for transmission.

In one specific embodiment, the monitor 300 has a barometric (absolute) pressure sensor, and a tare measurement is required to take into account variations in the ambient pressure from day to day or because of changes in altitude. This tare measurement could be taken the first time that the external device is present after an inhalation (e.g. at the same time as the data on the inhalation is transmitted to the external device), instead of immediately prior to inhalation if the external device is not present at that point.

## Claims

1. A monitor for an inhaler, wherein the monitor comprises one or more sensors, a microcontroller which is configured to process and/or store usage data determined from the sensor(s) relating to a patient's usage of the inhaler, a battery and a wireless communication unit having at least one antenna for transmitting the usage data to an external device via electromagnetic coupling, **characterized by** the wireless communication unit comprising an RFID controller communicatively coupled to the microcontroller via a synchronous serial communication bus for allowing the microcontroller to communicate the usage data to the RFID controller, the RFID controller having a memory for storing the received usage data for wireless transmission to the external device.

2. A monitor according to claim 1 wherein the RFID controller is arranged to harvest energy from the external device via the at least one antenna when the external device is electromagnetically coupled.

3. A monitor according to claim 2 wherein the RFID controller is arranged to provide at least a part of the harvested energy to the microcontroller.

4. A monitor according to claim 2 or claim 3 wherein the RFID controller is arranged to provide at least a part of the harvested energy to the battery.

5. A monitor according to any preceding claim wherein the microcontroller is configured to determine whether the external device is present, and, if the external device is present, to perform one or more further tasks whilst the usage data is being transmitted to the external device.

6. A monitor according to claim 5 wherein the one or more further tasks comprise compressing or trimming data before, during or after the data transfer.

7. A monitor according to claim 5 or claim 6 wherein the one or more further tasks comprise clearing the data in the memory of the RFID controller.

8. A monitor according to any of claims 5 to 7, wherein the one or more further tasks comprise cueing up usage data for subsequent transmission.

9. A monitor according to any of claims 5 to 8 comprising a barometric pressure sensor, wherein the one or more further tasks comprise taking a tare measurement from the pressure sensor.

10. A kit or a combination comprising a monitor according to any of claims 1 to 9 and an inhaler.

11. A kit or a combination according to claim 10 wherein the inhaler is a dry powder inhaler which contains a blister strip comprising a plurality of blisters.

12. A kit or a combination according to claim 10 or claim 11 wherein the monitor is removably attachable to the inhaler.

13. A combination according to claim 10 or claim 11 wherein the monitor is permanently attached to the inhaler.
